# EUROPEAN PATENT SPECIFICATION

(11) **EP 2 051 774 B1**
(45) Date of publication and mention of the grant of the patent: **12.09.2012**
(21) Application number: 07825922.3
(22) Date of filing: 17.07.2007
(51) Int. Cl.: A61N 5/06

(54) **SYSTEM OF PLASTER AND RADIATION DEVICE**
SYSTEM FÜR EINE PFLASTER- UND STRAHLUNGSVORRICHTUNG
SYSTEME DE PLATRE ET DISPOSITIF DE RAYONNEMENT

(30) Priority: 07.08.2006 EP 06118542
(43) Date of publication of application: 29.04.2009
(73) Proprietor: Koninklijke Philips Electronics N.V., 5621 BA Eindhoven (NL)
(72) Inventor: WAGENAAR CACCIOLA, Giovanna, NL-5656 AE Eindhoven (NL); HOELEN, Christoph, G., A., NL-5656 AE Eindhoven (NL); VAN AS, Marco, NL-5656 AE Eindhoven (NL)
(74) Representative: Bekkers, Joost J.J
(86) International application number: PCT/IB2007/052845
(87) International publication number: WO 2008/017975

(56) References cited:
- WO-A-00/50807
- WO-A-01/14012
- WO-A-02/100484
- WO-A-2007/126577
- DE-A1- 4 336 631
- US-A- 4 917 112
- US-A- 6 096 066

## Description

### FIELD OF THE INVENTION

The invention relates to a system of a plaster and a radiation device according to the preamble of claim 1.

The invention further relates to a plaster and to a radiation device suitable for use in such a system.

### BACKGROUND OF THE INVENTION

In a wound healing process three phases are generally distinguished, 1) an inflammation phase, when after injury the wound is covered by a cloth and bacteria are fought by the white blood cells; 2) a new tissue formation phase, when new-born tissue is made and the wound starts to contract, and 3) a tissue remodeling phase, when new-born tissue becomes mature scar tissue. It is known from literature that, in either one of the three phases, radiation therapy, for example light therapy, can be beneficial, i.e. in that it can accelerate and enhance the wound healing process in the first two phases and that it can keep the process under control in the last phase of tissue remodeling, counteracting overproduction of scar tissue thus leading to a nice scar.

A system is known from patent application WO02/100484. To ensure that the wound stays isolated from the environment and that the light emitting part does not get contaminated by bacteria coming from the wound or the wound does not get contaminated by bacteria coming from the radiation device, the system comprises said two mutually detachable parts. In the known system the plaster covers the wound, and physically separates the radiation device from the target area. Thus re-use of the radiation device is enabled by replacing or removing it from the plaster to exchange the light emitting part when desired or needed. The plaster comprises an adhesive surface that adheres to the skin, and an opening that leaves the target area, i.e. a skin having a wound, optically reachable by light. The radiation device is a light-emitting device which is (to be) placed over this opening, shining directly onto the target area. A disadvantage of the known system is that it provides a relatively uneven distribution of radiation over the target area. Another disadvantage of the known system is that it is of a rather spacious structure, which renders the construction less suitable for an application in a plaster as because of this the known system causes discomfort to a patient wearing the known system, and that it does enable continuous monitoring of the phase of the healing process.

WO 01/14012 discloses a flexible illuminator for external phototherapy having at least one light generating source, preferably a plurality of light generating sources, on a flexible substrate. Structure for diffusing light from the discrete light sources, and/or a system for transferring heat away from a skin contact surface are provided. The illuminator may be formed as a pad to be wrapped around an infant or limb of an adult, or as a mat.

### SUMMARY OF THE INVENTION

It is an object of the invention to provide a system of the opening paragraph in which the disadvantages are counteracted. Thereto the system of the opening paragraph is characterized by the characterizing portion of claim 1. To ensure that the target area is radiated uniformly, without the use of too many and too thick diffusing layers, the system is provided with an edge lit light guide that enables uniform distribution of the light over a predetermined surface area. Preferably, light extraction means are provided to extract the light from the light guide and preferably direct the light towards the target area. This is a construction analogous to a backlight construction as used, for example, in LCD computer screens. It is thus realized that the system is relatively compact and hence the wearability of the system by a human is improved. Preferably, the light-guide has an aspect ratio (of thickness to diameter or width) smaller than 0.2. Preferably, the system has a thickness-to-diameter aspect ratio smaller than 0.3. Suitable radiation sources that enable a further reduction in the proportions of the system are LEDs, organic LEDs (OLEDs), or laser diodes. It is thus enabled that a still more compact, integral system is obtained, which system is located in the vicinity of, i.e. only on or adjacent to, the target area thus further improving the wearability of the system.

The radiation device of the system preferably comprises a side-lit light-guide configuration with LED(s) mounted at an edge of the light-guide. In said construction the radiation, for example light having a wavelength of 630 nm, is first coupled into the light-guide using the total internal reflection, and finally homogeneously coupled out of the light-guide and supplied to the target area. It is thus realized that a relatively even distribution of light is obtained over the whole target area. In one embodiment, this window of the plaster is at least partially in optical contact with the light-guide of the radiation device, and thus optical contact causes a kind of light extraction from the light guide, i.e. by the optical contact, total internal reflection does not occur locally and radiation is transmitted into the window without a barrier from the light-guide and is subsequently delivered to the target area by light extraction means in or on the window of the plaster.

The window of the plaster is transparent or at least translucent for the radiation, in contact with the target area and preferably isolating the target area from the environment. It could, for example, be a conventional, known gel plaster, which diffuses the radiation and has a transmission in the visible region of ~ 50%. Alternatively, the window could be a foil as used in a Vacuum Assisted Closure (VAC) device. In the VAC a practically completely transparent foil is applied to the target area/wound and creates and maintains vacuum under the foil, keeping the wound isolated from the environment. The first part of the plaster could also be an ad hoc developed plaster with a specific light transmission in the visible region. The target area can be an injured part of a skin, wrinkles, acne, or a skin area suffering from diseases such as, for example, psoriasis, Vitiligo. The target area can be a part of a larger area to be treated, as can be the case with, for example, psoriasis, or can be limited to a local wound that is treated in its totality then.

To still further improve the wearability and comfort of the system, both the plaster and the light-guide should be made of a flexible material, e.g. silicone resin.

To increase the amount of radiation that is supplied to the target area, the system is characterized in that the light-guide is provided with radiation extraction means at the location of the window. Radiation extraction means could be, for example, scattering dots on an outer surface of the light-guide, a tapered shape, grooves, or scattering particles in the volume/bulk of the light-guide. It is thus enabled to deliver the radiation to the part covering the target area without the need for optical contact with the material of the window of the plaster. Here, "parts in optical contact" means that parts have a refractive index that is comparable, and that they are in direct, mutual contact such that there is no significant jump in refractive index for the radiation to pass, as would be the case if a layer of air would be found between two solid parts.

In another embodiment of the invention the system is characterized in that the light-guide is provided with a reflector at a side facing away from the target area. The light-guide provided with a reflector, reflects the radiation emitted by the LED and promotes the radiation to be directed towards the target area. This reflector could be a specularly reflective, for example, polymer foil provided with a metallic, for example aluminum, or a dichroic coating, or could alternatively be a diffuse reflective, for example, Teflon foil, depending on the desired distribution of radiation to be issued to the target area. In yet another embodiment the reflector is detachably mounted onto the radiation device to enable to adjust the spectrum or pattern of reflected radiation, the pattern, for example, being dependent on the phase of the healing process. In an alternative embodiment, the reflector is at least partially in optical contact with the light guide and comprises light extracting means. The reflector may comprise both diffuse and specularly reflective parts. In a special embodiment the at least partial optical contact is provided in a pattern to obtain a patterned light extraction.

In another embodiment of the invention the system is characterized in that the radiation extraction means and/or the reflector is provided in a patterned structure. Adjacent to/opposite to the window the light-guide is provided with radiation extracting features, for example diffuser dots, grooves, air bubbles, roughened surface, in order to obtain substantially homogeneous irradiation of the target area, i.e. skin/wound, in a predetermined area. Different radiation patterns could be achieved also when the light-guide is provided with a pattern of grooves or dots. Alternatively, the light-guide could be provided at a side facing, or opposite to, the target area with removable foils, possibly (partially) reflective, or could be provided with locally adapted/varying radiation extraction means, to modify the radiation pattern. Yet still alternatively, modification of the light extraction means is provided at the side of the light guide facing away from the target area. This feature can favorably be used in cases where parts of the target area need to be treated with different intensities of radiation. The light-guide and/or the reflector and the window are provided with a viewing window to enable continuous monitoring of the phase of the healing process.

In a preferred embodiment the system is characterized in that the radiation device comprises at least two radiation sources for the generation of at least two different radiation spectra. With the at least two types of LEDs emitting at substantially different wavelengths, for example UV-A radiation and red, a combination of different functions, for example disinfection and healing, can be issued simultaneously, UV-A radiation being radiation having a wavelength in the range of 320-400 nm.

In a preferred embodiment the system is characterized in that the radiation device supplies a radiation density of about 2.5 mW/cm² to the target area. In the case of about 50% transmission of the window, this means that approximately 5mW/cm² is to be emitted from the radiation device. A radiation density of 2.7 mW/cm² corresponding to an amount of energy of about 10 J/cm² provided in about 1 hour can accelerate the healing process. The embodiments according to the invention enable this accelerated healing process without the disadvantage of the target area drying up too much leading to a dry wound where the bacteria multiply more quickly. A radiation density of less than 2.5 mW, for example 1.0 mW, is applicable as well, but involves a relatively long time of the healing process.

In a preferred embodiment the system is characterized in that the radiation source is in a stacked location with respect to either the plaster coupling means or the window area. It is thus achieved that the system is kept as small/compact as possible. Hence, the comfort of the patient with respect to the wearability of the system is yet still further improved compared to known systems, and thus a risk of negative influence on the normal functioning of the patient is counteracted as much as possible. The same reasoning holds for the energy source. Therefore, a further preferred embodiment of the system is characterized in that the energy source comprises either a cell battery, flexible solar cells or a flexible battery pack in a stacked location with respect to the plaster coupling means and facing away from the substrate and plaster coupling means. Substrate includes for example skin tissue surface, either healthy skin tissue, infected skin tissue, damaged skin tissue, nail tissue, or hair tissue etc. The flexible battery could also be combined with the reflector foil. The system preferably comprises an electrical circuit for providing a regulated electrical current for the light source.

The invention is meant for a plaster application for wound healing, in particular for surgical wounds, to accelerate the wound healing process and to ensure that the scar looks nice after termination of the healing process, and for ulcers (non-healing wounds), like leg ulcers, diabetic ulcers, and decubitus ulcers. The plaster is also usable for treatment of wrinkles, possibly in combination with an anti-wrinkle creme, or is usable for disinfection of wounds, if UV (360 nm) or blue light (430 nm) is used. Furthermore, blue light (400-440 nm) is usable for local treatment of Acne (pimples) and UV radiation (312 nm) is usable for (local) treatment of Psoriasis and Vitiligo.

The invention further relates to a plaster and to a radiation device suitable for use in any system according to the invention.

US 6,096,066 discloses a flexible plaster which is provided with light sources mounted in an array covered with an optically transparent polymer material and with openings providing ventilation paths for air and moisture to move through the flexible substrate. The ventilation openings render the system relatively unprotected from the environment and bacteria. If a homogeneous illumination of the target area is desired, either a very high light source density is required which is expensive, or the system needs to be relatively thick, which is discomforting.

Furthermore, it is within the scope of this invention that the system is suitable for use in combination with medical emulsions, greases, balsams etc., and that the light-guide is optionally provided with one or more ventilation holes.

### BRIEF DESCRIPTION OF THE DRAWINGS

Further aspect of the invention will be further elucidated by means of the schematic drawing in which,
Fig. 1 shows a cross section of a system of a mutually detachable plaster and radiation device;
Fig. 2 shows a top view of the system of Fig. 1;
Fig. 3 shows a cross section of an embodiment of a system according to the invention;
Fig. 4 shows typical transmission curves for various types of windows of a translucent plaster.

### DETAILED DESCRIPTION OF EMBODIMENTS

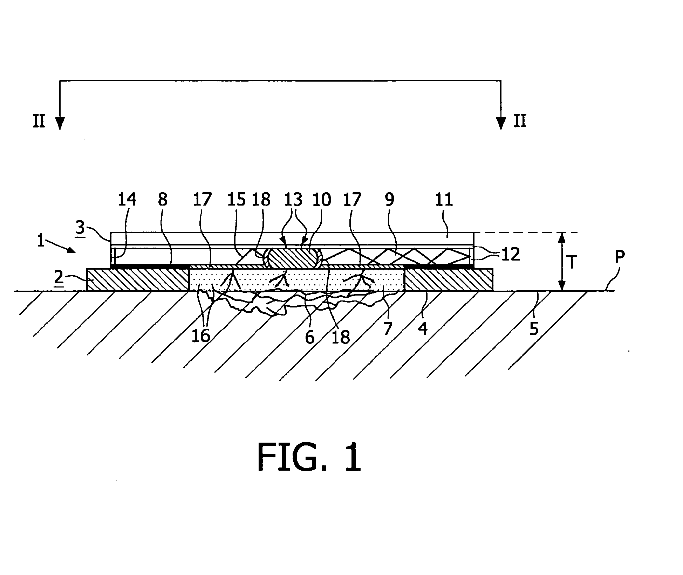

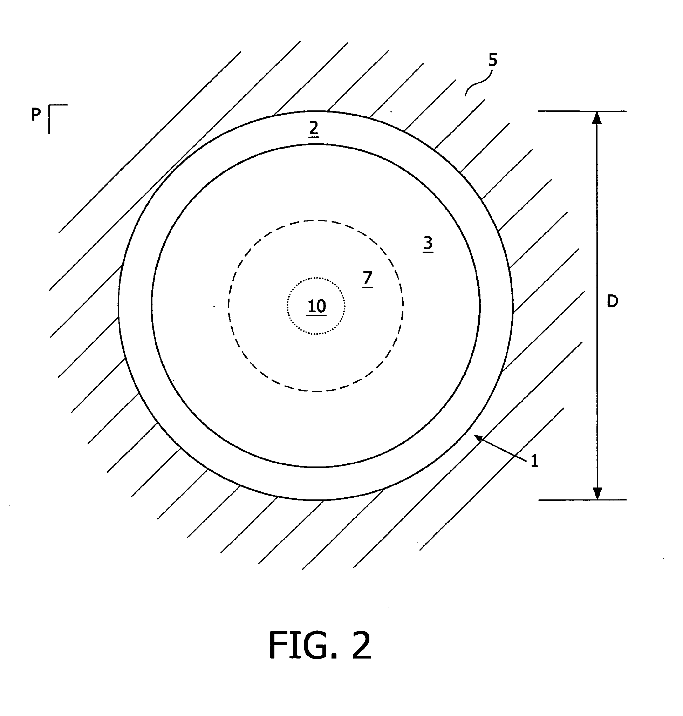

Fig. 1 shows a system 1 of a mutually detachable plaster and radiation device. The system 1 comprises a plaster 2 and in a stacked position, i.e. on top of the plaster, a radiation device 3 is adhered to the plaster via a first adhesive 8. The system 1 is attached via a second adhesive 4 to a skin surface 5 of a human, the skin surface 5 defines a plane P. The skin surface is damaged having caused a wound which wound defines a target area 6, being the outer, top surface area of the wound. The target area 6 is covered with a radiation transmissible window 7 of the plaster 2. The radiation device 3 comprises a light-guide 9 which is in optical contact with the window 7 via a coupling medium 17 for example an optically transparent or translucent adhesive, or a coupling gel, for example a silicone gel, and which is edge-lit by a radiation source 10. The radiation source and the light-guide are in mutually optical contact at edge 18, but optical contact is no requirement. The radiation source 10 is electrically connected via connectors 13 to a solar cell as the energy source 11, which is in stacked position relative to, i.e. is on top of, the light-guide 9, with a reflector 12, in the Fig. made of aluminum in between the solar cell and the light-guide 9. The reflector 12 further covers end faces 14 of the light-guide 9 and the part of the light-guide that is in contact with the adhesive 8, to prevent light extraction outside the area of the window 7. The radiation source generates radiation rays 15 which stay inside the light-guide 9 due to total internal reflection (TIR). In alternative embodiments this effect is obtainable by Fresnel reflections and/or reflection at reflective coatings. Because of multiple reflections a spreading of each of the radiation rays is obtained. When a ray impinges on scattering means 16 which are present in both bulk material and at the surface of the window, i.e. at an interface between the window 7 and the light-guide 9, the ray enters the window 7. In the bulk material of the window the ray is diffused due to the scattering means 16 in the bulk of the window and is subsequently diffusely issued onto the target area 6. In the figure the scattering means 16 comprise air/gas bubbles present in the silicone rubber material of the window. The system 1 has an average thickness T of about 5 mm and has a diameter D (see Fig.2) of about 30 mm, rendering the system to have a thickness to diameter ratio of less than 0.2. The top view, i.e. perpendicular to plane P as defined by the skin surface 5, of the system 1, shows a circular shaped plaster 2, with stacked on top of it the radiation device 3. For the sake of clarity, the positions of both the window 7 and the radiation source 10 are indicated by dotted lines.

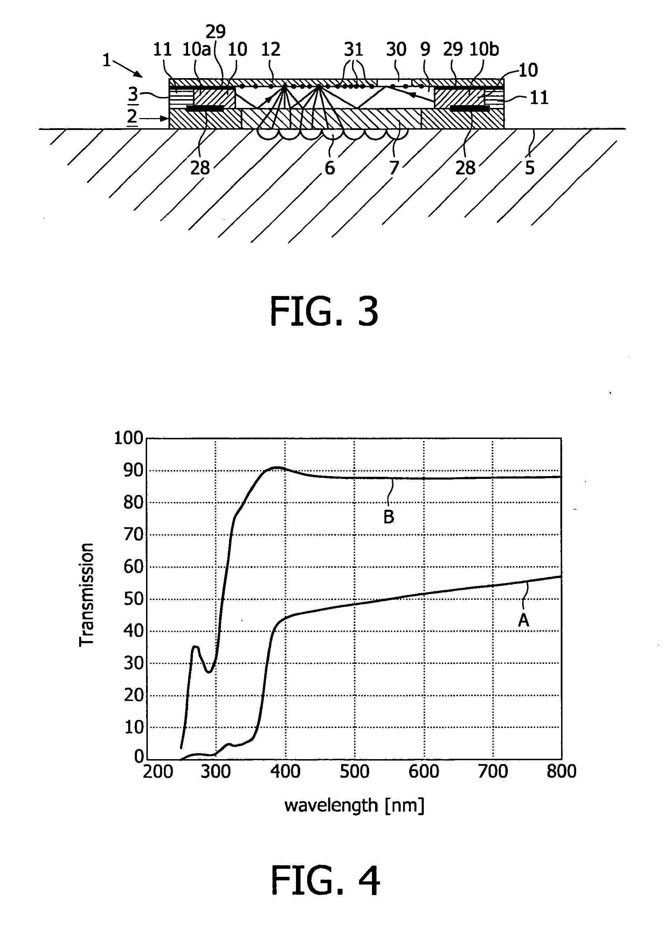

In Fig. 4 typical transmission curves for a translucent gel plaster window are shown, curves A and B for respectively the center of the window and near the edge of the window. The irradiation pattern/curve of a target area can thus be adjusted by selection of the window diffusive characteristics, too.

Fig. 3 shows a system 1 according to an embodiment of the invention, which system is adhered to the skin surface 5 of the skin of a human. The system 1 comprises a plaster 2 and in a stacked position, i.e. on top of the plaster, a radiation device 3 which is detachably connected to the plaster via Velcro 28. The radiation device comprises a cell battery as the energy source 11, electrically connected to a first LED 10a and a second LED 10b as the radiation sources 10. During operation the LEDs generate mutually different emission spectra, i.e. LED 10a generates UV-A radiation, i.e. radiation having a wavelength of about 360 nm, and LED 10b generates red light radiation having its peak wavelength in the range of about 610 to 660 nm. The LEDs are optically connected to a light-guide 9. A reflector 12, in the Fig. 3 a foil provided with a dichroic coating, is detachably stacked on top of the cell battery, the LEDs and the light-guide via an adhesive 29. The reflector is reflective for both visible and UV-A radiation, however, it is locally provided with a viewing window 30 which is transparent for visible radiation, thus enabling continuous monitoring of the healing process of the target area. Furthermore, the reflector is in optical contact with the light-guide and has a patterned structure as radiation extraction means 31. As shown in Fig. 3 the radiation extraction means are etched dots in the surface of the reflector facing the light-guide and are distributed over said surface with varying density, resulting in a desired, varying irradiation pattern of the target area. It is alternatively/additionally possible to optimize both the radiation profile of the LED and the shape of the edge of the light guide that accepts the light to achieve homogeneous illumination, in particular when the (diffuse) window of the plaster is used for the light extraction, for example to counteract that most of the light will be extracted at the location of or adjacent to the LED. The plaster is a VAC foil, which covers the damaged skin surface which is the target area 6. The VAC has a foil as the window 7 which is practically completely transparent for both visible radiation and UV-A. The VAC foil maintains vacuum between the damaged skin and the environment, thus keeping the wound isolated.

## Claims

1. A radiation device (3) stackable on and detachable from a plaster covering at least a target area and comprising:
plaster coupling means (4) for coupling the plaster to a substrate (5) adjacent to the target area, and
a window (7) for leaving the target area reachable by the radiation;
the radiation device comprising:
at least one radiation source (10) for generating radiation,
at least one energy source for supplying energy to the radiation source,
and transmission means (9) for guiding radiation towards a target area (6) wherein the transmission means comprises at least one radiation spreading light-guide (9), **characterized in that**
the light-guide (9) comprises a
viewing window (7, 30) for continuously monitoring the target area (6).

2. Device as claimed in claim 1, **characterized in that** the light-guide is flexible.

3. Device as claimed in claim 1 or 2, **characterized in that** the radiation device is flexible.

4. Device as claimed in claim 1 or 2, **characterized in that** the light-guide is provided with light extraction means (17) at the location of the window.

5. Device as claimed in claim 1 or 2, **characterized in that** the light guide is provided with diffusion means.

6. Device as claimed in claim 1 or 2, **characterized in that** the light-guide is provided with a reflector (12) at a side facing away from the target area.

7. Device as claimed in claim 1, **characterized in that** the radiation device comprises at least two radiation sources (10a, 10b) for the generation of at least two different emission spectra.

8. Device as claimed in any one of the previous claims, **characterized in that** the radiation device provides a radiation density of at least 2.5mW/cm2.

9. Device as claimed in claim 1 or 2, **characterized in that** the radiation device is detachable from the plaster while covering of the target area by the plaster is maintained.

10. Device as claimed in claim 1 or 2, **characterized in that** the radiation source is in a stacked location with respect to either the plaster coupling means or the window area.

11. Device as claimed in claim 1 or 2, **characterized in that** the radiation source comprises a semiconductor light-emitting device, e.g. a LED, an OLED and/or a laser diode.

12. Device as claimed in claim 1 or 2, **characterized in that** the radiation device comprises an electrical circuitry for providing a regulated current to the radiation source.

13. System (1) of stacked plaster (2) as defined in claim 1 and
radiation device (3) as claimed in any one of the previous mutually detachable.

14. System as claimed in claim 13, **characterized in that** the plaster comprises a Vacuum Assisted Closure foil as the window.

15. System as claimed in claim 13, **characterized in that** the plaster comprises a translucent gel or translucent rubber window.

## Patentansprüche

1. Strahlungsvorrichtung (3), die auf einem Pflaster stapelbar und hiervon abnehmbar ist, das mindestens einen Zielbereich abdeckt und Folgendes umfasst:
Pflasterkopplungsmittel (4) zum Koppeln des Pflasters mit einem an den Zielbereich angrenzenden Substrat (5), und
ein Fenster (7), um den Zielbereich durch die Strahlung erreichbar zu lassen, wobei die Strahlungsvorrichtung Folgendes umfasst:
mindestens eine Strahlungsquelle (10) zum Erzeugen von Strahlung,
mindestens eine Energiequelle zum Zuführen von Energie zu der Strahlungsquelle,
und Übertragungsmittel (9) zum Weiterleiten der Strahlung zu einem Zielbereich (6), wobei das Übertragungsmittel mindestens einen Strahlung streuenden Lichtleiter (9) umfasst,
**dadurch gekennzeichnet, dass**
der Lichtleiter (9) ein Sichtfenster (7, 30) zum kontinuierlichen Überwachen des Zielbereichs (6) umfasst.

2. Vorrichtung nach Anspruch 1, **dadurch gekennzeichnet, dass** der Lichtleiter flexibel ist.

3. Vorrichtung nach Anspruch 1 oder 2, **dadurch gekennzeichnet, dass** die Strahlungsvorrichtung flexibel ist.

4. Vorrichtung nach Anspruch 1 oder 2, **dadurch gekennzeichnet, dass** der Lichtleiter an dem Ort des Fensters mit Lichtextraktionsmitteln (17) ausgestattet ist.

5. Vorrichtung nach Anspruch 1 oder 2, **dadurch gekennzeichnet, dass** der Lichtleiter mit Diffusionsmitteln ausgestattet ist.

6. Vorrichtung nach Anspruch 1 oder 2, **dadurch gekennzeichnet, dass** der Lichtleiter auf der dem Zielbereich abgewandten Seite mit einem Reflektor (12) ausgestattet ist.

7. Vorrichtung nach Anspruch 1, **dadurch gekennzeichnet, dass** die Strahlungsvorrichtung mindestens zwei Strahlungsquellen (10a, 10b) zum Erzeugen von mindestens zwei verschiedenen Emissionsspektren umfasst.

8. Vorrichtung nach einem der vorhergehenden Ansprüche, **dadurch gekennzeichnet, dass** die Strahlungsvorrichtung eine Strahlungsdichte von mindestens 2,5 mW/cm² bereitstellt.

9. Vorrichtung nach Anspruch 1 oder 2, **dadurch gekennzeichnet, dass** die Strahlungsvorrichtung von dem Pflaster abnehmbar ist, während die Abdeckung des Zielbereichs durch das Pflaster erhalten bleibt.

10. Vorrichtung nach Anspruch 1 oder 2, **dadurch gekennzeichnet, dass** sich die Strahlungsquelle in einer gestapelten Position in Bezug auf entweder das Pflasterkopplungsmittel oder den Fensterbereich befindet.

11. Vorrichtung nach Anspruch 1 oder 2, **dadurch gekennzeichnet, dass** die Strahlungsquelle ein lichtemittierendes Halbleiterbauteil umfasst, zum Beispiel eine LED, eine OLED und/oder eine Laserdiode.

12. Vorrichtung nach Anspruch 1 oder 2, **dadurch gekennzeichnet, dass** die Strahlungsvorrichtung eine elektrische Schaltung zum Versorgen der Strahlungsquelle mit einem regulierten Strom umfasst.

13. System (1) aus gestapeltem Pflaster (2) nach Anspruch 1 und Strahlungsvorrichtung (3) nach einem der vorhergehenden Ansprüche, wobei das Pflaster (2) und die Strahlungsvorrichtung (3) voneinander abnehmbar sind.

14. System nach Anspruch 13, **dadurch gekennzeichnet, dass** das Pflaster eine Vakuumversiegelungsfolie als Fenster umfasst.

15. System nach Anspruch 13, **dadurch gekennzeichnet, dass** das Pflaster ein Fenster aus durchscheinendem Gel oder durchscheinendem Gummi umfasst.

## Revendications

1. Dispositif de rayonnement (3) empilable sur un pansement, et séparable de celui-ci, recouvrant au moins une zone cible et comprenant :
un moyen d'accouplement de pansement (4) pour accoupler le pansement avec un substrat (5) adjacent à la zone cible, et
une fenêtre (7) pour laisser la zone cible atteignable par le rayonnement ; le dispositif de rayonnement comprenant :
au moins une source de rayonnement (10) pour générer un rayonnement,
au moins une source d'énergie pour fournir de l'énergie à la source de rayonnement,
et un moyen de transmission (9) pour guider un rayonnement vers une zone cible (6), dans lequel le moyen de transmission comprend au moins un guide de lumière d'étalement de rayonnement (9),
**caractérisé en ce que**
le guide de lumière (9) comprend une fenêtre de visionnement (7, 30) pour surveiller la zone cible (6) en continu.

2. Dispositif selon la revendication 1, **caractérisé en ce que** le guide de lumière est flexible.

3. Dispositif selon la revendication 1 ou 2, **caractérisé en ce que** le dispositif de rayonnement est flexible.

4. Dispositif selon la revendication 1 ou 2, **caractérisé en ce que** le guide de lumière est pourvu d'un moyen d'extraction de lumière (17) à l'emplacement de la fenêtre.

5. Dispositif selon la revendication 1 ou 2, **caractérisé en ce que** le guide de lumière est pourvu d'un moyen de diffusion.

6. Dispositif selon la revendication 1 ou 2, **caractérisé en ce que** le guide de lumière est pourvu d'un réflecteur (12) sur un côté orienté dans une direction opposée à la zone cible.

7. Dispositif selon la revendication 1, **caractérisé en ce que** le dispositif de rayonnement comprend au moins deux sources de rayonnement (10a, 10b) pour la génération d'au moins deux spectres d'émission différents.

8. Dispositif selon une quelconque des revendications précédentes, **caractérisé en ce que** le dispositif de rayonnement fournit une densité de rayonnement d'au moins 2,5 mW/cm2.

9. Dispositif selon la revendication 1 ou 2, **caractérisé en ce que** le dispositif de rayonnement est séparable du pansement alors que la couverture de la zone cible par le pansement est maintenue.

10. Dispositif selon la revendication 1 ou 2, **caractérisé en ce que** la source de rayonnement est dans un emplacement empilé par rapport au moyen d'accouplement de pansement ou à la zone de fenêtre.

11. Dispositif selon la revendication 1 ou 2, **caractérisé en ce que** la source de rayonnement comprend un dispositif électroluminescent à semi-conducteur, par exemple une LED, une OLED et/ou une diode laser.

12. Dispositif selon la revendication 1 ou 2, **caractérisé en ce que** le dispositif de rayonnement comprend une circuiterie électrique pour fournir un courant régulé à la source de rayonnement.

13. Système (1) de pansement (2) selon la revendication 1 et de dispositif de rayonnement (3) selon une quelconque des revendications précédentes empilés, le pansement (2) et le dispositif de rayonnement (3) étant mutuellement séparables.

14. Système selon la revendication 13, **caractérisé en ce que** le pansement comprend une feuille de fermeture assistée par vide en tant que fenêtre.

15. Système selon la revendication 13, **caractérisé en ce que** le pansement comprend un gel translucide ou une fenêtre en caoutchouc translucide.
